# EUROPEAN PATENT SPECIFICATION

(11) **EP 3 634 206 B1**
(45) Date of publication and mention of the grant of the patent: **11.08.2021**
(21) Application number: 18739655.1
(22) Date of filing: 04.06.2018
(51) Int. Cl.: A61B 5/00, A61B 5/053, A61B 5/0536, A61B 5/0205, A61B 5/0537, A61B 5/282

(54) **MULTIFUNCTIONAL DEVICE FOR REMOTE MONITORING OF A PATIENT'S CONDITION**
MULTIFUNKTIONALE VORRICHTUNG ZUR FERNÜBERWACHUNG EINES PATIENTENZUSTANDS
DISPOSITIF MULTIFONCTIONNEL PERMETTANT DE SURVEILLER À DISTANCE L'ÉTAT D'UN PATIENT

(30) Priority: 05.06.2017 PL 42179917
(43) Date of publication of application: 15.04.2020
(73) Proprietor: Telemedical Innovations, 44-100 Gliwice (PL)
(72) Inventor: PLAZA, Ireneusz, 41-909 Bytom (PL); KONARSKI, Lukasz, 54-440 Wroclaw (PL); CACKO, Andrzej, 05-300 Minsk Mazowiecki (PL)
(74) Representative: Lukaszyk, Szymon
(86) International application number: PCT/PL2018/000057
(87) International publication number: WO 2018/226108

(56) References cited:
- CN-A- 103 315 722
- GB-A- 2 425 181
- US-A- 5 341 806
- US-A- 5 445 149
- US-A1- 2008 287 770
- US-A1- 2011 288 605
- US-A1- 2013 225 985
- US-B1- 8 019 402
- US-B1- 9 192 313
- US-B1- 9 579 055

## Description

The invention is as specified in the claims.

The present disclosure relates to a multifunctional device for remote monitoring of a patient's condition, in particular the patient's heart electrical activity and the level of hydration of the patient's chest cavity, having a form of a harness adapted to be worn on the patient's body at the patient's torso area, comprising electrocardiograph (ECG) electrodes, bioimpedance electrodes, a central unit processing signals originating from said electrodes and a communication module for a wireless transmission of signals processed by said central unit to a remote monitoring system.

Patent specification US4966154 discloses a system for monitoring a patient comprising a harness in a form of three belts joined together at the front and at the rear of the patient; a horizontal belt which detachably encircles the chest of a patient, and two shoulder belts joined together to the horizontal belt. The harness incorporates an ECG data sensing device to produce first and second analog voltages which are measurements of the body MCL1 and MCL6 data respectively; a respiratory amplitude sensing device; and a temperature sensing device. Respiratory data is obtained by measuring the change in impedance between the two respiratory electrodes disposed in the horizontal belt at the front and at the rear of the patient. This harness does not allow however to conduct a full-featured ECG measurement, that is a measurement in 12 lead placement Likar-Mason system due to the lack of a belt embracing the patient's torso at the pelvis area.

Patent specification GB2458389 discloses a harness comprising various sensors measuring patient data, such as ECG, respiratory frequency, e.g. by means of impedance measurements, skin surface temperature, as well as body movements, e.g. by means of an accelerometer.

Publication WO 2016/097921 discloses a harness for measuring a patient's respiratory response, as well as complete ECG measurement in the Likar-Mason system. The respiratory response measurements are conducted by means of an assembly of accelerometers detecting changes of positions of the patient's chest during respiratory movements. Although this harness enables to measure ECG in the Likar-Mason system, it does not enable to measure respiratory response by means of an electrical bioimpedance analysis.

Harnesses or belts adapted to be worn on a patient's body that enable join simplified ECG measurement, as well as bioelectric impedance analysis are also disclosed in publications US9326695, WO2016149829, US2013225985 and US2014330142.

It has been an object of the present disclosure to provide a multifunctional device for remote monitoring of a patient's condition enabling simultaneous ECG analysis according to the 12 lead Likar-Mason system and the level of hydration of the patient's chest cavity (including lungs) by means of an electrical bioimpedance analysis.

The disclosure provides a multifunctional device for remote monitoring of a patient's condition described at the outset, characterized in that, said bioimpedance electrodes comprise front electrodes disposed at the front of the patient's body and rear electrodes corresponding to the front electrodes and disposed at the back of the patient's body, arranged in pairs, wherein at least one pair of said bioimpedance electrodes defines a line crossing through the left part of the patient's chest and at least one pair of said bioimpedance electrodes defines a line crossing through the right part of the patient's chest.

Such an arrangement of bioimpedance electrodes ensures independent measurement of the level of hydration of the left and the right part of the patient's chest cavity, hence also left and right lung lobe, and therefore improves efficiency of the monitoring of the patient's condition.

Preferably the device comprises many pairs of said bioimpedance electrodes defining lines crossing through the left part of the patient's chest and many pairs of said bioimpedance electrodes defining lines crossing through the right part of the patient's chest.

A large number (for example eight pairs) of bioimpedance electrodes ensures the measurement of not only the level of hydration but also diagnosis employing Electrical Impedance Tomography (EIT).

Preferably said harness comprises a horizontal belt, embracing the patient's torso in the epigastric region and above the pelvis, connected in the breastbone area and the back of the patient with a first vertical belt and a second vertical belt, that extend over the patient's right and the left shoulder girdles, wherein said horizontal belt is equipped with precordial ECG electrodes and leg ECG electrodes, while said first and said second vertical belt are equipped with precordial ECG electrodes and arm ECG electrodes.

Such a construction enables 12 lead ECG measurement in the Likar-Mason system. Preferably said horizontal belt is connected at the area of the patient's back with said first and said second vertical belt by means of a coupler.

The coupler stabilizes the construction of the harness.

In such a case preferably said right and left front bioimpedance electrodes are disposed at the front section of said first and second vertical belt and at the front section of said horizontal belt, while right and left rear bioimpedance electrodes are disposed in the coupler.

This enables broad spectrum of measurements both through upper and lower areas of the lungs.

Preferably the device of the present disclosure comprises at least one additional sensor selected form the group containing a temperature sensor, a humidity sensor and an accelerometer.

Preferably said ECG electrodes and said bioimpedance electrodes have a form of dry pin electrodes.

Electrodes of this type are easy to clean.

Preferably said harness is made of elastomeric material, such as synthetic rubber or silicone.

Materials of this type enable to embed the electronic components of the device in the material volume, thanks to that the harness neatly adjoins the body and is waterproof.

Preferably said harness is made as one element.

Therefore it may be easy cut out of a base sheet.

Preferably said harness material comprises silver nanoparticles.

Silver nanoparticles feature strong antibacterial, antifungal and antiviral properties.

They release slowly from the matrix of material they are embedded by ionic exchange and interfere microorganism cellular functions.

The disclosure is described below in an exemplary embodiment and illustrated in the drawing, in which:
Fig. 1 shows a patient with a device according to the disclosure attached on the patient's chest in a front view (Fig. 1a); rear view (Fig. 1b) and side view (Fig. 1c);
fig. 2 shows the device shown in Fig. 1 in a front perspective view;
fig. 3 shows the device shown in Fig. 1 before being attached to a patient's torso; and
Fig. 4 schematically illustrates a monitoring system comprising many devices of the present disclosure.

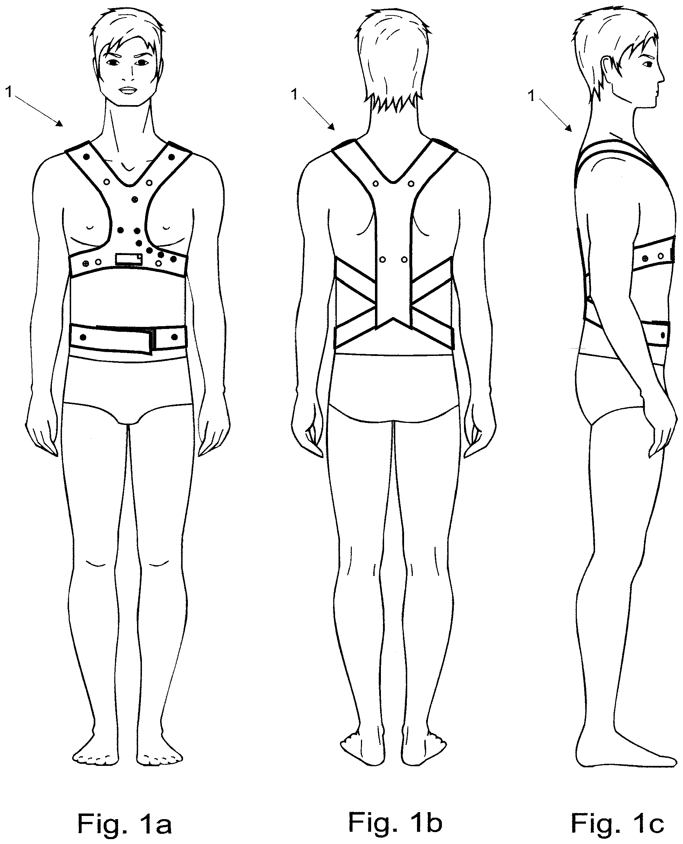

A multifunctional device for remote monitoring of a patient's condition is shown in Fig. 1 after being attached on the patient's torso. Although in this embodiment monitored patient is human, the device may obviously be used with appropriate adjustments for remote monitoring of an animal's, for example a dog's, condition.

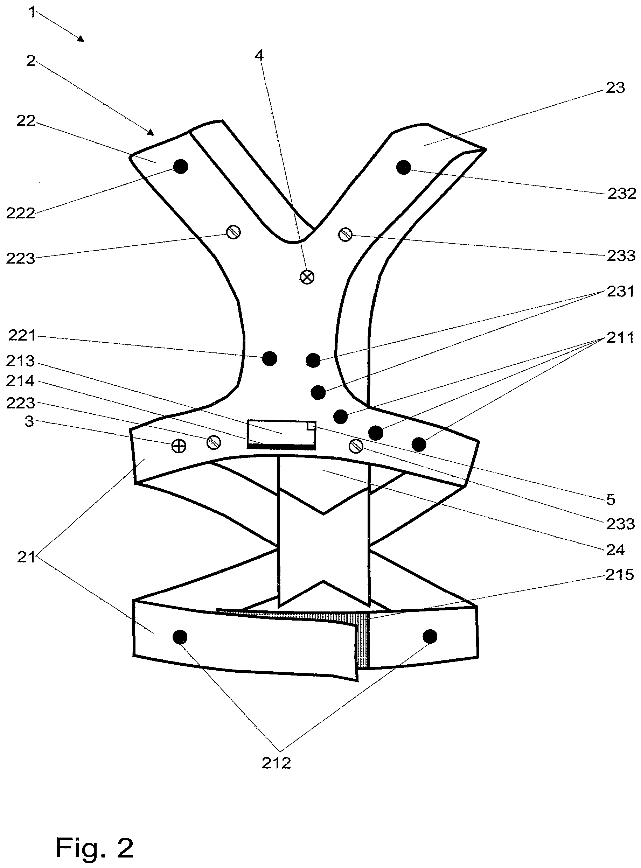

As shown in Fig. 2, the device 1 comprises a harness 2 in a form of three belts: a horizontal belt 21 and two vertical belts; right belt 22 and left belt 23. Material of the harness 2 is in this embodiment neoprene comprising silver nanoparticles. The harness 2 may be made however from any other material adapted to adjoin a patient's skin, such as for example COOLMAX® freshFX™.

The horizontal belt 21 embraces patient's torso twice; first around the epigastric region and subsequently below, around the pelvis. Vertical belts 22, 23 extend over the right and the left shoulder girdles and are connected with the horizontal belt 21 at the front of the torso in the area of the breastbone and at the back of the torso by means of a coupler 24 with orifice, the functionality of which shall be described later.

The harness 2 comprises a twelve lead placement Likar-Mason electrocardiograph measurement system comprising six precordial electrodes 221, 231, 211 (V1, V2, V3, V4, V5, V6) disposed in standard locations and four limb electrodes 212, 222, 232 disposed in standard locations for arms (RA, LA) and legs (RL, LL).

Precordial ECG electrodes 211 (V4, V5, V6) and leg ECG electrodes 212 (RL, LL) are disposed in the horizontal belt 21. The horizontal belt 21 also comprises an adjusting element 215 enabling to change the circumference of the horizontal belt 21 depending on the patient's dimensions. In the presented embodiment, the adjusting element 215 has a form of a Hookhand-loop fastener (Velcro®). It is obvious however that other fasteners such as buckles, buttons or various latches may be used.

Precordial ECG electrode 221 (V1) disposed in the right vertical belt 22, while precordial ECG electrodes 231 (V2, V3) are disposed in the left vertical belt 23.

The device 1 furthermore comprises a temperature sensor 3 and a humidity sensor 4. In the presented embodiment the temperature sensor 3 is disposed in the horizontal belt 21 in the patient's epigastric region, while the humidity sensor 4 is disposed in the left vertical belt 23. These sensors may obviously be disposed in any other part of the harness 2, both at the horizontal belt 21, as well as at any of the vertical belts 22, 23.

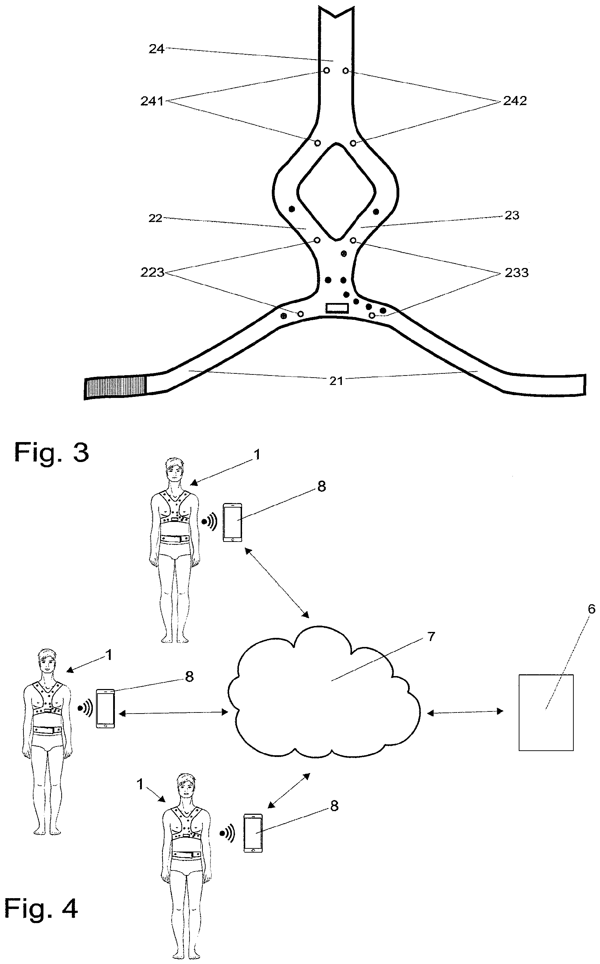

As shown in Fig. 2 and Fig. 3, the harness 2 also comprises electrical bioimpedance measurement system comprising four pairs of electrodes: two pairs of bioimpedance electrodes 223, 241 at the right and two pairs of bioimpedance electrodes 233, 242 at the left side of the patient's chest. This enables to assess the level of hydration of tissues between the electrodes, as well as the changes of this level on the basis of an analysis of an electrical resistance (impedance) of the tissues.

Right front bioimpedance electrodes 223 are disposed at the right vertical belt 22, while left front bioimpedance electrodes 233 are disposed at the left vertical belt 23 at the front of the patient's torso. Bioimpedance electrodes 223, 233 are also disposed at the horizontal belt 21. Right rear bioimpedance electrodes 241 and left rear bioimpedance electrodes 242 are disposed at the coupler 24 at the back of the patient's torso. A pair of bioimpedance electrodes 223, 241 defines a line crossing through a right patient's lung lobe, while another pair of bioimpedance electrodes 233, 242 defines a line crossing through a left patient's lung lobe.

Electrical bioimpedance of the right half of the chest is measured between electrodes 223 and electrodes 241. Analogously bioimpedance of the right left of the chest is measured between electrodes 233 and electrodes 242. To this end a flow of current of a relatively small amperage (in the range of 1-10 µA) is induced between the first bioimpedance electrode selected as transmitting and the second bioimpedance electrode selected as receiving and voltage between these electrodes is measured. A large number of such measurements enables to approximate the cavity of the patient's chest using Electrical Impedance Tomography (EIT) medical imaging.

Bottom bioimpedance electrodes 241, 242 are disposed at the coupler 24, in such a way that after the device 1 is attached and tied they are located at approximately the same height as the bottom bioimpedance electrodes 223, 233.

In this embodiment all the electrodes are dry pin electrodes. It is obvious however to use classic electrodes, ball electrodes, silicon-carbon electrodes, etc.

Central unit 213 is furthermore disposed at the horizontal belt 21 to process the signals of the ECG electrodes 211, 212, 221, 222, 231, 232, bioimpedance electrodes 223, 233, 241, 242 and sensors 3, 4. An accelerometer 5 is an additional component of the central unit 213 and serves to measure the position and movements of the patient's body, ensuring assessment of the patient's physical activity and body movements that may influence measurements. The accelerometer 5 also measures the time of using the device, which may be used e.g. for tele-rehabilitation purposes. The accelerometer 5 may also switch the device 1 on and off.

After performing a few (at least three) measurements from bioimpedance electrodes 223, 233, 241, 242, the central unit 213 may calculate their moving average (updated with each new measurement) and on this basis compute a confidence interval and a standard deviation. If any new measurement does not fall within the calculated confidence interval, a signal of a fluid in the left or right lung lobe may be generated.

Signals processed by the central unit 213 are transmitted to a communication module 214, which may be wirelessly connected, for example using a Bluetooth transmission protocol with a patient's communication device 8, for example a smartphone, tablet or personal computer with an appropriate processing application installed.

Monitoring system comprising many devices 1 according to the invention is schematically illustrated in Fig. 4. As shown each patient is wearing a device 1, the communication module of which communicates with the patient's mobile communication device 8, and by means of the communication device 8, via computer network 7 with a remote monitoring system 6 which may operate automatically or be supervised by a professional physician or a group of physicians.

In other embodiments of the disclosure, the harness may not comprise a coupler at the spinal area, bioimpedance electrodes 241, 242 may be disposed at the first and at the second vertical belt 22, 23 at the back area, vertical belts 22 and 23 may be directly connected from both sides to the horizontal belt 21, etc.

Furthermore, the communication module 214 itself may comprise means (such as a GSM module with a SIM card) enabling direct wireless transmission of signals processed by the central unit 213 to a remote monitoring system 6 with no need of using patient's communication device 8. The device 1 according to the disclosure may also comprise other modules, not illustrated in the drawing, such as battery module, accumulator module charged using electrical induction, etc.

The device 1 according to the disclosure may also be used to conduct a classical resting ECG measurement, as well as to monitor atrial fibrillation and chronic heart failure, stable ischemic heart disease, cardiac arrhythmia, in telemonitoring and cardiac telerehabilitation, etc.

The figures are not necessarily to scale, and some features may be exaggerated/minimized, in order to provide better illustration. Therefore the presented embodiments should not be regarded as limiting the intended scope of protection which is defined in the patent claims.

### List of reference numerals

1. multifunctional device for remote monitoring of a patient's condition
2. harness
   21. horizontal belt
      211. precordial electrocardiograph electrode (V4, V5, V6)
      212. leg electrocardiograph electrode (RL, LL)
      213. central unit
      214. communication module
      215. adjusting element
   22. right vertical belt
      221. precordial electrocardiograph electrode (V1)
      222. right arm electrocardiograph electrode (RA)
      223. right front bioimpedance electrode
   23. left vertical belt
      231. precordial electrocardiograph electrode (V2, V3)
      232. left arm electrode (LA)
      233. left front bioimpedance electrode
   24. coupler
      241. right rear bioimpedance electrode
      242. left rear bioimpedance electrode
3. temperature sensor
4. humidity sensor
5. accelerometer
6. remote monitoring system
7. computer network
8. communication device

## Claims

1. A multifunctional device (1) for remote monitoring of a patient's condition, in particular the patient's heart electrical activity and the level of hydration of the patient's chest cavity, having a form of a harness (2) adapted to be worn on the patient's body at the patient's torso area, comprising electrocardiograph (ECG) electrodes (211, 212, 221, 222, 231, 232), bioimpedance electrodes (223, 233, 241, 242), a central unit (213) processing signals originating from said electrodes and a communication module (214) for a wireless transmission of the signals processed by said central unit (213) to a remote monitoring system (6), **characterized in that**, said bioimpedance electrodes (223, 241; 233, 242) comprise front electrodes (223, 233) disposed at the front of the patient's body and rear electrodes (241, 242) corresponding to the front electrodes (223, 233) and disposed at the back of the patient's body, arranged in pairs, wherein
it comprises many pairs of said bioimpedance electrodes (223, 241) defining lines crossing through the left part of the patient's chest and many pairs of said bioimpedance electrodes (233, 242) defining lines crossing through the right part of the patient's chest, wherein
said harness (2) comprises a horizontal belt (21), embracing the patient's torso in the epigastric region and above the pelvis, connected in the breastbone area and the back of the patient with a first vertical belt (22) and a second vertical belt (23), that extend over the patient's right and left shoulder girdles, wherein said horizontal belt (21) is connected at the area of the patient's back with said first and said second vertical belt (22, 23) by means of a coupler (24) and
said right and left front bioimpedance electrodes (223, 233) are disposed at the front section of said first and second vertical belt (22, 23) and at the front section of said horizontal belt (21), while right and left rear bioimpedance electrodes (241, 242) are disposed in the coupler (24).

2. The device according to Claim 1, **characterized in that**, said horizontal belt (21) is equipped with precordial ECG electrodes (211) and leg ECG electrodes (212), while said first and said second vertical belt (22, 23) are equipped with precordial ECG electrodes (221, 231) and arm ECG electrodes (222, 232).

3. The device according to any one of the preceding Claims, **characterized in that**, it comprises at least one additional sensor selected form the group containing a temperature sensor (3), a humidity sensor (4) and an accelerometer (5).

4. The device according to any one of the preceding Claims, **characterized in that**, said harness (2) is made of elastomeric material, such as synthetic rubber or silicone.

5. The device according to any one of the preceding Claims, **characterized in that**, said harness (2) is made as one element.

6. The device according to any one of the preceding Claims, **characterized in that**, said harness (2) material comprises silver nanoparticles.

## Patentansprüche

1. Ein multifunktionales Gerät (1) zur Fernüberwachung des Zustands eines Patienten, insbesondere der elektrischen Herzaktivität des Patienten und des Hydratationsniveaus der Brusthöhle des Patienten, die die Form eines Gurtzeuges (2) aufweist, das dazu geeignet ist, am Körper des Patienten im Bereich des Rumpfes des Patienten getragen zu werden, und das Elektrokardiograph/EKG-Elektroden (211, 212, 221, 222, 231, 232), Bioimpedanzelektroden (223, 233, 241, 242), eine Zentraleinheit (213), die von den Elektroden stammende Signale verarbeitet, und ein Kommunikationsmodul (214) für eine drahtlose Übertragung der von der Zentraleinheit (213) verarbeiteten Signale an ein Fernüberwachungssystem (6) umfasst, **dadurch gekennzeichnet, dass** die Bioimpedanzelektroden (223, 241; 233, 242) vordere Elektroden (223, 233), die an der Vorderseite des Körpers des Patienten angeordnet sind, und hintere Elektroden (241, 242), die den vorderen Elektroden (223, 233) entsprechen und an der Rückseite des Körpers des Patienten angeordnet sind, umfassen, und in Paaren angeordnet sind, wobei
es viele Paare der Bioimpedanzelektroden (223, 241) umfasst, die Linien definieren, die durch den linken Teil der Brust des Patienten verlaufen, und viele Paare der Bioimpedanzelektroden (233, 242), die Linien definieren, die durch den rechten Teil der Brust des Patienten verlaufen, wobei
das Gurtzeug (2) einen horizontalen Gurt (21) umfasst, der den Rumpf des Patienten im epigastrischen Bereich und oberhalb des Beckens umschließt und im Bereich des Brustbeins und des Rückens des Patienten mit einem ersten vertikalen Gurt (22) und einem zweiten vertikalen Gurt (23) verbunden ist, die sich über den rechten und linken Schultergürtel des Patienten erstrecken, wobei
der horizontale Gurt (21) im Bereich des Rückens des Patienten mit dem ersten und dem zweiten vertikalen Gurt (22, 23) mittels eines Verbindungsstückes (24) verbunden ist und
die rechte und linke vordere Bioimpedanzelektroden (223, 233) am vorderen Abschnitt des ersten und zweiten vertikalen Gurtes (22, 23) und am vorderen Abschnitt des horizontalen Gurtes (21) angeordnet sind, während die rechte und linke hintere Bioimpedanzelektroden (241, 242) im Verbindungsstück (24) angeordnet sind.

2. Das Gerät nach Anspruch 1, **dadurch gekennzeichnet, dass** der horizontale Gurt (21) mit präkordialen EKG-Elektroden (211) und Bein-EKG-Elektroden (212) ausgestattet ist, während der erste und der zweite vertikale Gurt (22, 23) mit präkordialen EKG-Elektroden (221, 231) und Arm-EKG-Elektroden (222, 232) ausgestattet sind.

3. Das Gerät nach einem der vorhergehenden Ansprüche, **dadurch gekennzeichnet, dass** es mindestens einen zusätzlichen Sensor umfasst, der aus der Gruppe ausgewählt ist, die einen Temperatursensor (3), einen Feuchtigkeitssensor (4) und einen Beschleunigungsmesser (5) enthält.

4. Das Gerät nach einem der vorhergehenden Ansprüche, **dadurch gekennzeichnet, dass** der Gurt (2) aus elastomerem Material, wie synthetischem Kautschuk oder Silikon, hergestellt ist.

5. Das Gerät nach einem der vorhergehenden Ansprüche, **dadurch gekennzeichnet, dass** das Gurtzeug (2) als ein Element ausgeführt ist.

6. Das Gerät nach einem der vorhergehenden Ansprüche, **dadurch gekennzeichnet, dass** das Material des Gurtzeuges (2) Silber-Nanopartikel umfasst.

## Revendications

1. Un dispositif multifonctionnel (1) pour la surveillance à distance de l'état d'un patient, en particulier l'activité électrique cardiaque du patient et le niveau d'hydratation de la cavité thoracique du patient, ayant la forme d'un harnais (2) adapté pour être porté sur le corps du patient au niveau de la zone du torse du patient, comprenant des électrodes d'électrocardiographe (ECG) (211, 212, 221, 222, 231, 232), des électrodes de bioimpédance (223, 233, 241, 242), une unité centrale (213) traitant des signaux provenant desdites électrodes et un module de communication (214) pour une transmission sans fil des signaux traités par ladite unité centrale (213) à un système de surveillance à distance (6), **caractérisé en ce que**, lesdites électrodes de bioimpédance (223, 241; 233, 242) comprennent des électrodes avant (223, 233) disposées à l'avant du corps du patient et des électrodes arrière (241, 242) correspondant aux électrodes avant (223, 233) et disposées à l'arrière du corps du patient, disposées par paires, dans lequel
il comprend plusieurs paires desdites électrodes de bioimpédance (223, 241) définissant des lignes traversant la partie gauche de la poitrine du patient et plusieurs paires desdites électrodes de bioimpédance (233, 242) définissant des lignes traversant la partie droite de la poitrine du patient, dans lequel ledit harnais (2) comprend une ceinture horizontale (21), embrassant le torse du patient dans la région épigastrique et au-dessus du bassin, reliée dans la zone du sternum et le dos du patient avec une première ceinture verticale (22) et une seconde ceinture verticale (23), qui s'étendent sur les ceintures scapulaires droite et gauche du patient, dans lequel
ladite ceinture horizontale (21) est reliée à la zone du dos du patient avec lesdites première et seconde ceintures verticales (22, 23) au moyen d'un coupleur (24) et lesdites électrodes de bioimpédance (223, 233) avant droite et gauche sont disposées à la section avant desdites première et seconde ceintures verticales (22, 23) et à la section avant de ladite ceinture horizontale (21), tandis que les électrodes de bioimpédance arrière droite et gauche (241, 242) sont disposées dans le coupleur (24).

2. Le dispositif selon la revendication 1, **caractérisé en ce que** ladite ceinture horizontale (21) est équipée d'électrodes ECG précordiales (211) et d'électrodes ECG de jambe (212), tandis que ladite première et ladite seconde ceinture verticale (22, 23) sont équipées d'électrodes ECG précordiales (221, 231) et d'électrodes ECG de bras (222, 232).

3. Le dispositif selon l'une quelconque des revendications précédentes, **caractérisé en ce qu'**il comprend au moins un capteur supplémentaire choisi dans le groupe comprenant un capteur de température (3), un capteur d'humidité (4) et un accéléromètre (5).

4. Le dispositif selon l'une quelconque des revendications précédentes, **caractérisé en ce que** ledit harnais (2) est réalisé en matériau élastomère, tel que du caoutchouc synthétique ou du silicone.

5. Le dispositif selon l'une quelconque des revendications précédentes, **caractérisé en ce que** ledit harnais (2) est réalisé en un seul élément.

6. Le dispositif selon l'une quelconque des revendications précédentes, **caractérisé en ce que** ledit matériau de harnais (2) comprend des nanoparticules d'argent.
